# EUROPEAN PATENT APPLICATION

(11) **EP 1 916 246 A2**
(43) Date of publication of application: **30.04.2008**
(21) Application number: 07019763.7
(22) Date of filing: 10.10.2007
(51) Int. Cl.: C07D 403/10, C07D 405/14

(54) **An improved process for the preparation of olmesartan medoxomil**

(30) Priority: 11.10.2006 IN MU16712006
(71) Applicant: Cadila Pharmaceuticals Limited, Bhat, Ahmedabad 382 210 (IN)
(72) Inventor: Modi, Indravadan Ambalal, Ahmedabad - 382 210 (IN); Bagepalli, Chandrasekhar Sujatha, Ahmedabad - 382 210 (IN); Gurusamy, Renugadevi, Ahmedabad - 382 210 (IN); Ponnaiah, Ravi, Ahmedabad - 382 210 (IN); Khamar, Bakulesh Mafatial, Ahmedabad - 382 210 (IN)
(74) Representative: Towler, Philip Dean

(57) **Abstract**

Olmesartan medoxomil of high purity (99.3-99.7% by HPLC ) is prepared using an improved process of its intermediate, namely- ethyl-4-(1-hydroxy-1-methylethyl)-2-propyl-1-[[2'-(2-(triphenylmethyl)-2H-tetrazol-5yl]biphenyl-4-yl]methyl]imidazole-5-carboxylate, comprising:
Reacting ethyl-4-(1-hydroxy-1-methylethyl)-2-propylimidazole-5-carboxylate with N-(Triphenylmethyl)-5-[4'-(bromomethyl)biphenyl-2- yl]tetrazole in an organic solvent in presence of a base and a phase transfer catalyst in non-aqueous system to give after workup, ethyl-4-(1-hydroxy-1-methylethyl)-2-propyl-1-[[2'-[2-(triphenylmethyl)-2H-tetrazol-5yl]biphenyl-4-yl]methyl]imidazole-5-carboxylate, which is further processed, by following improved reaction conditions in three steps to provide substantially pure [HPLC purity 99.3 to 99.7 %] olmesartan medoxomil.
A further process relates to the purification of olmesartan medoxomil by treatment with isopropyl alcohol and methyl ethyl ketone.

## Description

### FIELD OF THE INVENTION

The present invention relates to an improved process for the preparation of ethyl-4-(1-hydroxy-1-methylethyl) 2-propyl- 1-[[2'-[2-(triphenylmethyl)-2H-tetrazol-5yl]biphenyl-4-yl]methyl]imidazole -5-carboxylate which is an intermediate in the preparation of Olmesartan medoxomil.

### BACKGROUND OF THE INVENTION

Olmesartan medoxomil is known by two names,
(a)(5-Methyl-2-oxo-1,3-dioxolen-4-yl)methyl 4-(1-hydroxy-1-methylethyl)-2-propyl-1-[4-(2(tetrazole-5yl)phenyl]phenyl]methylimidazole-5-carboxylate
(b)4-(1-Hydroxy-1-methylethyl)-2-propyl-1-[[2'-(1H-tetrazol-5-yl)[1,1'-biphenyl]-4-yl]methyl]-1H-imidazol-5-carboxylic acid (5-methyl-2-oxo-1,-3 dioxol-4-yl)methyl ester, and has a CAS No. [144689-63-4].

The structural formula is represented below:

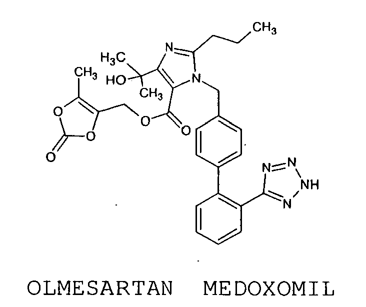

The prior art synthetic methods involve coupling reaction between the substituted imidazole and substituted biphenyl methyl bromide. J. Med. Chem. 1996 vol. 39 No.1, page 323-38 describes the synthesis of Olmesartan medoxomil as follow. [Scheme-1 ]

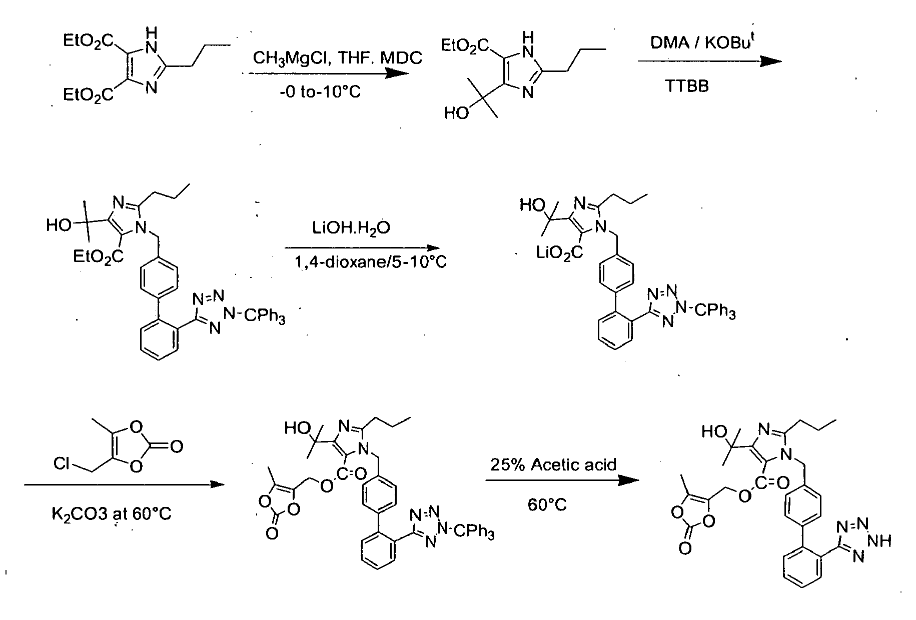

US 5616599 describes a process for the preparation of olmesartan medoxomil as follows.

4-(1-hydroxyl-1-methylethyl)-2-propyl imidazole-5carboxylic acid is reacted with 5-methyl-2-oxo-1, 3-dioxolene-4-yl)methyl chloride using N,N-diisopropylethyl amine as base in N,N-dimethyl acetamide at 60°C to give (5-methyl-2-oxo-1,3-dioxolen-4-yl)methyl4-(1-hydroxy-1-methylethyl)-2-propyl imidazole-5-carboxylate. The resulting product is coupled with N-(triphenylmethyl)-5-[4'-(bromomethyl)biphenyl-2-yl]tetrazole [herein referred to as TTBB] at 60°C in N, N-dimethyl acetamide using potassium carbonate as base to give protected olmesartan medoxomil. The protected olmesartan medoxomil is deprotected using 75 % acetic acid to give olmesartan medoxomil.

This process involves column chromatographic purification of intermediates which is not desirable on commercial scale operation.

US 5616599 describes another process for the preparation of olmesartan Medoxomil which involves addition of methyl Magnesium chloride on diethyl 2-propylimidazole-4, 5-dicarboxylate in tetrahydrofuran at -30 to -20°C to give ethyl-4-(1-hydroxy-1-methylethyl)-2-propylimidazole -5-carboxylate, which is coupled with TTBB using sodium hydride as base in N, N-dimethylformamide at 60°C to give ethyl-4-(1-hydroxy-1-methylethyl)2-propyl-1-[[2'-[2-(triphenylmethyl)-2H-tetrazol-. 5yl]biphenyl-4-yl]methyl]imidazole-5-carboxylate. The product thus formed is hydrolyzed using lithium hydroxide monohydrate as base in 1,4-dioxane at 5-10°C to give lithium salt of 4-(1-hydroxy-1-methylethyl)-2-propyl-1-[[2'-[2-(triphenylmethyl)-2H-tetrazol-5yl]biphenyl-4-yl]methyl]imidazole-5-carboxylic acid, which is then coupled with 5-methyl-2-oxo-(1,3-dioxolene-4-yl)methyl chloride using K₂CO₃ as base in N,N-dimethylacetamide at 50°C to give trityl protected olmesartan medoxomil which on deprotection using 75% acetic acid gives Olmesartan Medoxomil.

During the condensation of ethyl-4-(1-hydroxy-1-methylethyl)-2-propylimidazole -5-carboxylate, with TTBB using sodium hydride as base in N, N-dimethylformamide, various impurities are formed, and isolation of the product involves extractive workup.

It is a long-felt need to provide improved process that can be performed on a commercial scale, without the need for chromatographic purification, does not use carcinogenic solvent and multi-step extractive workup to give olmesartan medoxomil in high purity [> 99.5% by HPLC]

### SUMMARY OF THE INVENTION:

The main object of the present invention is to provide an improved process for the commercial production of Olmesartan medoxomil.

Another object of the invention is to provide a process without use of chromatographic purification.

Yet another object of the invention is to provide an alternative process for the synthesis of an intermediate for the synthesis of olmesartan medoxomil.

Yet another object of the invention is to provide a process that reduces the levels of impurities in each stage and give olmesartan medoxomil of HPLC purity >99.5 %.

The present invention provides an improved process for the preparation of ethyl-4-(1-hydroxy-1-methylethyl)-2-propyl-1-[[2'-[2-(triphenylmethyl)-2H-tetrazol-5yl] biphenyl-4-yl]methyl]imidazole - 5-carboxylate by coupling reaction between Ethyl-4-(1-hydroxy-1-methylethyl)-2-propylimidazole -5-carboxylate and TTBB in an organic solvents such as THF, MTBE, MeCN, toluene, acetone, MIBK, heptane ,preferably MIBK, MTBE and THF using a base such as carbonates and bicarbonates, preferably carbonates such as anhydrous potassium carbonate and a phase transfer catalyst in non-aqueous system.

### DETAILED DESCRIPTION OF THE INVENTION

In accordance with the present invention, the synthesis of olmesartan medoxomil comprises:
[1] Reacting diethyl-2-propyl imidazole-4,5-dicarboxylate with methyl magnesium halide in an organic solvent to give after workup, ethyl-4-(1-hydroxy-1-methylethyl)-2-propylimidazole -5-carboxylate,
[2] reacting ethyl-4-(1-hydroxy-1-methylethyl)-2-propylimidazole -5-carboxylate with N-(triphenylmethyl)-5-[4'-(bromomethyl)biphenyl-2-yl]tetrazole in an organic solvent, such as THF, MTBE, MeCN, toluene, acetone, MIBK, heptane, preferably MIBK, MTBE, and THF and using a base such as carbonates and bicarbonates of alkali metal, preferably anhydrous potassium carbonate and phase transfer catalyst in non-aqueous system to give after workup, ethyl-4-(1-hydroxy-1-methylethyl) 2-propyl- 1-[[2'-[2-(triphenylmethyl)-2H-tetrazol-5yl]biphenyl-4-yl]methyl]imidazole -5-carboxylate,
[3] reacting ethyl-4-(1-hydroxy-1-methylethyl) 2-propyl- 1-[[2'-[2-(triphenylmethyl)-2H-tetrazol-5yl]biphenyl-4-yl]methyl]imidazole -5-carboxylate in an organic solvent [excluding 1,4-dioxane] with LiOH.H2O to give after work-up, Lithium salt of 4-(1-hydroxy-1-methylethyl)-2-propyl-1-[[2'-[2-(triphenylmethyl)-2H-tetrazol-5yl]biphenyl-4-yl]methyl]imidazole -5-carboxylic acid,
[4] reacting the lithium salt of 4-(1-hydroxy-1-methylethyl)-2-propyl-1-[[2'-[2-(triphenylmethyl)-2H-tetrazol-5yl]biphenyl-4-yl]methyl]imidazole-5-carboxylic acid in an organic solvent with 5-methyl-2-oxo-(1,3-dioxolene-4-yl)methyl chloride; in presence of organic tertiary amine base to give after workup, trityl protected olmesartan medoxomil,
[5] reacting trityl protected olmesartan medoxomil with aqueous acetic acid to give olmesartan medoxomil, which is further, crystallized using isopropyl alcohol followed by purification from methyl ethyl ketone to give substantially pure [HPLC purity 99.3 to 99.7 %] olmesartan medoxomil.

### For step- [1]:

Methyl magnesium halide used for the reaction is methyl magnesium chloride or methyl magnesium bromide or methyl magnesium iodide.

Solvent used for this step is selected from ethers such as THF, 2-Methyl THF, 1,2-dimethoxy ethane, dibutyl ether, aromatic hydrocarbon solvents such as toluene, xylene or mixtures thereof; the preferred solvent is THF.

The temperature for the reaction is from about -20 to 45°C preferably from -15 to 25°C. After the reaction is over compound is isolated by extractive workup.

### For step-[2]:

The base used for the coupling is selected from carbonates such as anhydrous Na₂CO₃, K₂CO₃, Li₂CO₃, Cs₂CO₃, BaCO₃,CaCO₃ and bicarbonates such as, NaHCO₃,KHCO₃; preferred base being anhydrous potassium carbonate.

The solvent used for the reaction is selected from ethers such as THF, MTBE, di-n-propyl ether or mixtures thereof, the preferred solvents being MTBE,THF; nitriles such as acetonitrile, propionitrile;; ketonic solvents such as acetone, methyl ethyl ketone, methyl isobutyl ketone, or mixtures thereof; the preferred solvents being acetone, methyl ethyl ketone , methyl isobutyl ketone. The reaction is carried out at 0 to 150°C, preferably at 55 to 100°C.

Phase transfer catalyst used for the reaction is selected from:
(a) Quaternary ammonium salts such as N(R₁R₂R₃R₄)X wherein R₁,R₂,R₃, and R₄ is C₁ to C₁₃ alkyl or aralkyl group, cycloalkyl, aryl, or heterocyclyl, X being a monovalent anion .The preferred catalyst is triethylbenzylammonium chloride or tetrabutyl ammoniun bromide or tetrabutylammonium hydrogen sulfate; the more preferred catalyst being tetrabutylammoniun bromide,

### For step- [3]:

The solvent used for the reaction is selected from ether group of solvents such as THF, 2- methyl THF, methyl tert. butyl ether, monoglyme and their like or mixtures thereof; the preferred solvent being THF.

The reaction is carried out at -10 to +50°C, preferably below 10°C for addition of base and at 20 to 30°C after the addition of base. The material obtained after extractive work up is isolated as such and is carried forward for the next step.

### For step- [4]:

The solvent used for the reaction is selected from polar aprotic solvents such as DMF, DMA, NMP and DMSO, ketonic solvents such as acetone, MEK, MIBK or mixtures thereof. The preferred solvent is DMA.

The base used for the condensation is selected from:
tertiary amines such as triethyl amine, N-methyl morpholine, diisopropyl ethyl amine, the preferred base being triethyl amine.

The reaction is carried out at -20°C to 100°C preferably below 10°C during addition and 25 to 100°C for completing the reaction after the addition is over. The reaction is worked up using aqueous quenching followed by layer separation, distillation and treating the residue with methanol.

### For step- [5]:

For deprotecting trityl olmesartan medoxomil, the requirement is the reaction of this compound with an acid preferably an aqueous acid.

The acid used for trityl deprotection is selected from aq. H₂SO₄, aqueous hydrochloric acid, aqueous phosphoric acid, aqueous acetic acid, aqueous formic acid and their like, preferably aqueous acetic acid.

The temperature range selected for the deprotection is from 0 to 100°C, preferably between 35 to 80°C.

The compound is then crystallized from 2-propanol, which is further purified using methyl ethyl ketone.

The present invention does not involve chromatographic purification at any stage of synthesis, suppresses impurity formation observed in step-2 , avoids use of carcinogenic solvent such as dioxane , uses modified reaction conditions in selecting reagents and solvents in steps 4 and 5, to give substantially pure [HPLC purity 99.3 to 99.7 %] olmesartan medoxomil.

The present invention is illustrated with following examples, which do not limit the scope of the invention.

### Example-1

### Preparation of Ethyl-4-(1-hydroxy-1-methylethyl)-2-propylimidazole-5-carboxylate

To a 3M solution of MeMgCl(55.86 g, 0.74 mol) in tetrahydrofuran was added a solution of diethyl 2-propyl imidazole- 4,5-dicarboxylate (50 g,0.19 mol) in tetrahydrofuran (200 ml) at -10 to 0°C under N₂ atmosphere. The mixture was stirred at -5 to 0°C for 10 minutes. Reaction mass was quenched into 400 ml 25 % ammonium chloride solution followed by extraction with ethyl acetate (300 ml). The organic phase was separated, washed with brine, dried over Na₂SO₄, and concentrated in vacuo to give a syrup, which was crystallized using diisopropyl ether.
Yield: 85-90 %,
Purity by HPLC: 88-93 %.
1H-NMR (CDCl₃) δ: 7.8-8.1 (s, 1H), 5.8(s, 1H)., 4.35(q, 2H), 2.68(t, 2H), 1.78(m, 2H), 1.61(s, 6H), 1.36(t, 3H), 0.96(t, 3H).

### Example-2

### Preparation of Ethyl-4-(1-hydroxy-1-methylethyl)-2-propyl-1-[[2'-[2-(triphenylaiethyl)-2H-tetrazol-5yl]biphenyl-4-yl]methyl]imidazole -5-carboxylate

Mixture of Ethyl-4-(1-hydroxy-1-methylethyl)-2-propylimidazole -5-carboxylate (41 g, 0.17 mol), potassium carbonate (47g, 0.34 mol) and tetrabutylammonium bromide (4.9 g, 0.01 mol) in acetone was stirred at room temperature for 1hr. Then TTBB (93% Purity, 92.89g, 0.15 mol) was charged, refluxed for 14hrs. Potassium salts were filtered off from the reaction mass and the filtrate was charcoalised for 1hr. It was filtered over celite bed and the filtrate was distilled off completely to get a semi solid mass. 250 ml of Methanol was added to the residue and stirred for 2-3 hrs to give a solid product, which was filtered and washed with chilled methanol and dried.
Yield: 80-85%,
Purity by HPLC: 85-90%.
1H-NMR (CDCl₃) δ: 7.8-8.1 (m, 1H), 6.7-7.61 (m, 22H), 5.78 (bs, 1H), 5.38(s, 2H), 4.12 (q, 2H), 2.52 (t, 2H), 1.64(s, 6H), 1.5-1.8(m, 2H), 1.08(t, 3H), 0.88(t, 3H).

### Example-3

### Preparation of lithium salt of 4-(1-hydroxy-1-methylethyl) 2-propyl-1-[[2'-[2-(triphenylmethyl)-2H-tetrazol-5yl] biphenyl-4-yl] methyl] imidazole -5-carboxylic acid

To a solution of Ethyl-4-(1-hydroxy-1-methylethyl) 2-propyl- 1-[[2'-[2-(triphenylmethyl)-2H-tetrazol-5yl]biphenyl-4-yl]methyl]imidazole-5-carboxylate (105 g, 0.14 mol) in tetrahydrofuran , was added LiOH.H₂O (7.8 g, 0.18 mol) solution below 10°C. The reaction mixture was stirred at room temperature for 15 hours. Reaction mass was concentrated under vacuum at 35°C to 1/4 th of its volume. 300 ml of ethyl acetate and NaCl (130 g) were added to the residue under stirring. The organic phase was separated, dried over sodium sulphate and concentrated under vacuum to get the product. The crude product was taken as such to the next stage.

### Example-4

### Preparation of trityl protected olmesartan medoxomil

To the solution of lithium salt of 4-(1-hydroxy-1-methylethyl) 2-propyl- 1-[[2'-[2-(triphenylmethyl)-2H-tetrazol-5yl] biphenyl-4-yl] methyl] imidazole -5-carboxylic acid , (97 g, 0.13 mol) in N, N-dimethyl acetamide(200 ml) was added triethylamine(12.7 g, 0.12 mol), stirred at room temperature for 0.5 hours. 5-methyl-2-oxo-(1,3-dioxolene-4-yl)methyl chloride (85% purity, 37.3 g, 0.25 mol) was added below 10°C. The mixture was stirred at 50-55°C for 4 hours, checked TLC. Dichloromethane (400 ml) and chilled water (500 ml) were added under stirring. The organic phase was separated, given brine wash (50 ml), dried over sodium sulphate and concentrated under vacuum to get a residue. To the residue methanol was added, stirred for 1hr, cooled to 5-10°, filtered and washed with chilled methanol and dried.
Yield: 75-80%,
Purity by HPLC: 96-98%.
1H-NMR (CDCl₃) δ: 7.87(d, 1H), 6.90-7.52(m, 20H), 6.68(d, 2H), 5.61(s, 1H), 5.3(s, 2H), 4.7(s, 2H), 2.54(t, 2H), 1.97(s, 3H), 1.6-1.75(m, 2H), 1.62(s, 6H), 0.87(t, 3H).

### Example-5

### Preparation of olmesartan medoxomil

To the suspension of trityl protected olmesartan medoxomil (50g, 0.06 mol) in 250 ml 75% acetic acid was stirred at 50-55°C for 1.5hrs and cooled to 5-10°C. The by-product trityl alcohol was filtered off and washed with 75% acetic acid. The filtrate was concentrated under vacuum to get syrup, which was crystallized using isopropyl alcohol.
Yield: 85-88%,
Purity by HPLC: 95-98%.
The material was further purified with ethyl methyl ketone. It was filtered and washed with ethyl methyl ketone and dried to give substantially pure olmesartan medoxomil.
Yield: 70-75%,
Purity by HPLC: 99.3-99.7%.
1H-NMR (CDCl₃) δ: 7.81(dd, 1H), 7.43-7.6(m, 3H), 7.09d, 2H), 6.79(d, 2H), 5.41(s, 1H),4.95(s, 1H), 2.56(t, 3H), 2.17(s, 3H), 1.58-1.69(m, 2H), 1.58(s, 6H), 0.92(t,3H).

## Claims

1. A process for the preparation of substantially pure olmesartan medoxomil in steps comprising:
[a] Reacting ethyl-4-(1-hydroxy-1-methylethyl)-2-propylimidazole -5-carboxylate with N-(Triphenylmethyl)-5-[4'-(bromomethyl)biphenyl-2-yl]tetrazole in an organic solvent in presence of a base and a phase transfer catalyst in non-aqueous system to give ethyl-4-(1-hydroxy-1-methylethyl) 2-propyl- 1-[[2'-[2-(triphenylmethyl)-2H-tetrazol-5yl]biphenyl-4-yl]methyl]imidazole -5-carboxylate,
[b] reacting ethyl-4-(1-hydroxy-1-methylethyl) 2-propyl-1-[[2'-[2-(triphenylmethyl)-2H-tetrazol-5yl]biphenyl-4-yl]methyl]imidazole -5-carboxylate in an organic solvent [excluding 1,4-dioxane] with LiOH.H20 to give Lithium salt of 4-(1-hydroxy-1-methylethyl)-2-propyl-1-[[2'-[2-(triphenylmethyl)-2H-tetrazol-5yl]biphenyl-4-yl]methyl]imidazole -5-carboxylic acid ,
[c] reacting Lithium salt of 4-(1-hydroxy-1-methylethyl)-2-propyl-1-[[2'-[2-(triphenylmethyl)-2H-tetrazol-5yl]biphenyl-4-yl]methyl]imidazole-5-carboxylic acid in an organic solvent with 5-methyl-2-oxo-(1,3-dioxolene-4-yl)methyl chloride using an organic tertiary amine base to give trityl protected olmesartan medoxomil,
[d] reacting trityl protected olmesartan medoxomil with aqueous acetic acid to give olmesartan medoxomil, which is further, crystallized using isopropyl alcohol followed by purification from methyl ethyl ketone to give substantially pure olmesartan medoxomil.

2. The process of claim-1 wherein the organic solvent in step-[a] is selected from ethers such as THF, MTBE, di-n-propyl ether or mixtures thereof; nitriles such as acetonitrile, propionitrile; aliphatic and alicyclic solvents such as hexane, heptane, cyclohexane, methyl cyclohexane or mixtures thereof; ketonic solvents such as acetone, methyl ethyl ketone, methyl isobutyl ketone, or mixtures thereof.

3. The process of claim-2 wherein the preferred organic solvents are THF,MTBE, MeCN, toluene, acetone, methyl isobutyl ketone heptane ; more preferred solvents being MIBK, MTBE, and THF.

4. The process of claim-1 wherein a base used in step-[a] is selected from carbonates and bicarbonates such as anhydrous Na₂CO₃, K₂CO₃, Li₂CO₃, Cs₂CO₃, NaHCO₃, KHCO₃, BaCO₃, CaCO₃ or mixtures thereof.

5. The process of claim-1 wherein the base used in step[a] is preferably anhydrous potassium carbonate.

6. The process of claim-1 wherein a phase transfer catalyst used in step-[a] is selected from:
Quaternary ammonium salts such as N(R₁R₂R₃R₄)X, wherein R₁,R₂,R₃, and R₄ is C₁ to C₁₃ alkyl or aralkyl group, cycloalkyl, aryl, or heterocyclyl, X being monovalent anion ,

7. The process of claim-6 wherein the phase transfer catalyst used in step[a] is triethylbenzylammonium chloride or tetrabutyl ammoniun bromide, or tetrabutyl ammonium hydrogen sulfate; the more preferred catalyst being tetrabutylammoniun bromide.

8. The process of claim-1 wherein the reaction in step-[a] is carried out at temperature ranging from about 40 to 150°C.

9. The process of claim-1 wherein the reaction in step-[a] is preferably carried out at 55-100°C.

10. The process of claim-1 wherein the product recovered after filtration and solvent removal in step-[a] is purified in C1-C4 alcohols.

11. The process of claim-10 wherein the preferred alcohol is methanol.

12. The process of claim-1 wherein the reaction in step-[b] is carried out at -10 to +50°C

13. The process of claim-1 wherein the reaction in step-[b] is preferably carried out below 10°C for addition of base and at 20 to 30°C after the addition of base.

14. The process of claim-1 wherein the solvent used in step-[b] is selected from ether group of solvents such as THF, 2-methyl THF, methyl tert. butyl ether, monoglyme or mixtures thereof.

15. The process of claim-1 wherein the preferred solvent used in step-[b] is THF.

16. The process of claim-1 wherein the solvent used in step-[c] is selected from N,N-dialkyl amide group of solvents such as DMF,DMA,NMP; DMSO, ketonic solvents such as acetone, MEK, MIBK or mixtures thereof.

17. The process of claim-1 wherein the preferred solvent used in step-[c] is N,N-dimethylacetamide.

18. The process of claim-1 wherein the reaction in step-[c]is carried out at -20°C to 100°C.

19. The process of claim-1 wherein the reaction in step-[c] is preferably carried out below 10°C during addition and at 25 to 100°C for completing the reaction after the addition is over.

20. The process of claim-1 wherein the organic tertiary amine base used in step-[c] is selected from triethyl amine, N-methyl morpholine, diisopropyl ethyl amine.

21. The process of claim-20 wherein the preferred base is triethyl amine.

22. The process of claim-1 wherein the product obtained in step-[d], after trityl deprotection, is further, crystallized using isopropyl alcohol followed by purification from methyl ethyl ketone to give substantially pure [HPLC purity 99.3 to 99.7 %] olmesartan medoxomil.

23. Purification of olmesartan medoxomil comprising treatment with isopropyl alcohol , methyl ethyl ketone to give substantially pure [HPLC purity 99.3 to 99.7 %] olmesartan medoxomil.

24. Reacting ethyl-4-(1-hydroxy-1-methylethyl)-2-propylimidazole -5-carboxylate with N-(Triphenylmethyl)-5-[4'-(bromomethyl)biphenyl-2-yl]tetrazole in an organic solvent in presence of a base and a phase transfer catalyst in non-aqueous system to give ethyl-4-(1-hydroxy-1-methylethyl) 2-propyl- 1-[[2'-[2-(triphenylmethyl)-2H-tetrazol-5y1]biphenyl-4-yl]methyl]imidazole -5-carboxylate.
